# EUROPEAN PATENT SPECIFICATION

(11) **EP 0 326 384 B1**
(45) Date of publication and mention of the grant of the patent: **29.09.1993**
(21) Application number: 89300756.7
(22) Date of filing: 26.01.1989
(51) Int. Cl.: A61B 5/02, A61B 5/021

(54) **Pulse wave detecting apparatus**
Pulswellenmessgerät
Dispositif pour détecter l'onde à impulsion

(30) Priority: 29.01.1988 JP 11974/88 U
(43) Date of publication of application: 02.08.1989
(73) Proprietor: COLIN CORPORATION, Komaki-shi Aichi 485 (JP)
(72) Inventor: Kawamura, Norio, 2007-1 Hayashi Komaki-shi Aichi-ken (JP); Kaida, Noriyuki, 2007-1 Hayashi Komaki-shi Aichi-ken (JP)
(74) Representative: Senior, Alan Murray

(56) References cited:
- EP-A- 0 122 123
- DE-A- 2 737 665
- DE-A- 3 008 601
- US-A- 4 269 193

## Description

The present invention relates to improvements in a pulse wave detecting apparatus.

There has been proposed a pulse wave detecting apparatus having a pressing means for pressing an arterial vessel located near a body surface of a subject, for example a radial artery near a wrist of the subject, the pressing means being fixed on the body surface such that the pressing means is aligned with the arterial vessel. The pulse wave detecting apparatus has a pulse wave detecting means for detecting pulse waves of the arterial vessel which are produced by expansion and contraction of the vessel synchronized with heart beat of the subject as a result of the pressing of the pressing means. Further, the apparatus is provided with a fixing means for fixing the pressing means to the body surface over the arterial vessel, for example a band having at both ends thereof a pair of engaging members such as a pair of slide fasteners. With the band wound around a body portion of the subject, for example a wrist of the subject, and fastened by the engaging members, the pressing means is fixed in place over the arterial vessel of the subject.

Such a pulse wave detecting apparatus is disclosed for example in US-A-4,269,193.

However, it is considerably time-consuming to fix the pressing means on the body surface over the arterial vessel by means of the band or other similar fixing means.

It is therefore an object of the present invention to provide a pulse wave detecting apparatus wherein the housing thereof accommodating the pressing means thereof is more easily fixed to a body surface over an arterial vessel.

The above object has been achieved by the present invention, which provides a pulse wave detecting apparatus having
a housing which is to be fixed to a body surface of a subject,
pressing means accommodated in said housing, for pressing an artery of said subject via said body surface, and
pulse wave detecting means for detecting pulse waves of said artery which are produced in relation with the pressing of said pressing means,
the apparatus including,
an adhesive member mounted on said housing such that said adhesive member is located around said pressing means accommodated in said housing, said adhesive member adhering said housing to said body surface of said subject with an adhesive force higher than the pressing force of said pressing means.

In the pulse wave detecting apparatus constructed as described above, the adhesive member mounted on the housing is adhered to around the body surface right above the artery. Subsequently the pressing means is activated to press the artery, and the produced pulse wave is detected by the pulse wave detecting means. Thus, the housing and accordingly the pressing means accommodated therein are easily fixed in place on the body surface over the artery by simply adhering to the body surface the adhesive member secured to the housing. Thus, the present pulse wave detecting apparatus is more easily handled than the conventional apparatus of the above-mentioned type.

In a preferred embodiment of the pulse wave detecting apparatus of the present invention, the pressing means includes a diaphragm which is inflated by fluid pressure applied thereto, and the pulse wave detecting means consists of a pressure sensor which is accommodated in the housing and is pressed against the body surface by the inflated diaphragm so as to detect pressure variation produced at an interface between the pressure sensor and the body surface, the detected pressure variation representing the pulse wave produced in relation with the pressing of the diaphragm.

In another embodiment of the apparatus of the invention, the pressing means consists of a diaphragm which is inflated by fluid pressure applied thereto, and the pulse wave detecting means detects pressure variation produced in a space defined by at least the diaphragm, the detected pressure variation representing the pulse wave. In this embodiment, the pulse wave detecting means may be accommodated in the housing. Alternatively, the pulse wave detecting means may be located outside the housing, and connected to the housing via a pipe.

In yet another embodiment of the apparatus of the invention, the pressing means consists of a bag which is inflated by fluid pressure applied thereto, and the pulse-wave detecting means detects pressure variation in the bag, the detected pressure variation representing the pulse wave.

In a further embodiment of the apparatus of the invention, the housing is formed of synthetic resin and has a generally disk-like configuration with a comparatively shallow cavity, and the adhesive member consists of an adhesive sheet fixed to the disk-like housing along a circumference of the housing, the adhesive sheet being adhered to the body surface with the adhesive force.

By way of example, the present invention will be better understood by reading the following detailed description of the presently preferred embodiment of the invention, when considered in conjunction with the accomapying drawings, in which:
Fig. 1 is an illustrative view showing a pulse wave detecting apparatus of the present invention;
Fig. 2 is a perspective view showing a pulse wave detecting probe of the apparatus of Fig. 1;
Figs. 3 through 5 are cross-sectional views of other pulse wave detecting probes which are employed in the apparatus of Fig. 1; and
Figs. 6 and 7 are cross-sectional views partly showing other adhesive sheets which are empolyed in the apparatus of Fig. 1.

### DETAILED DESCRIPTION OF THE PREFERRED EMBODIMENT

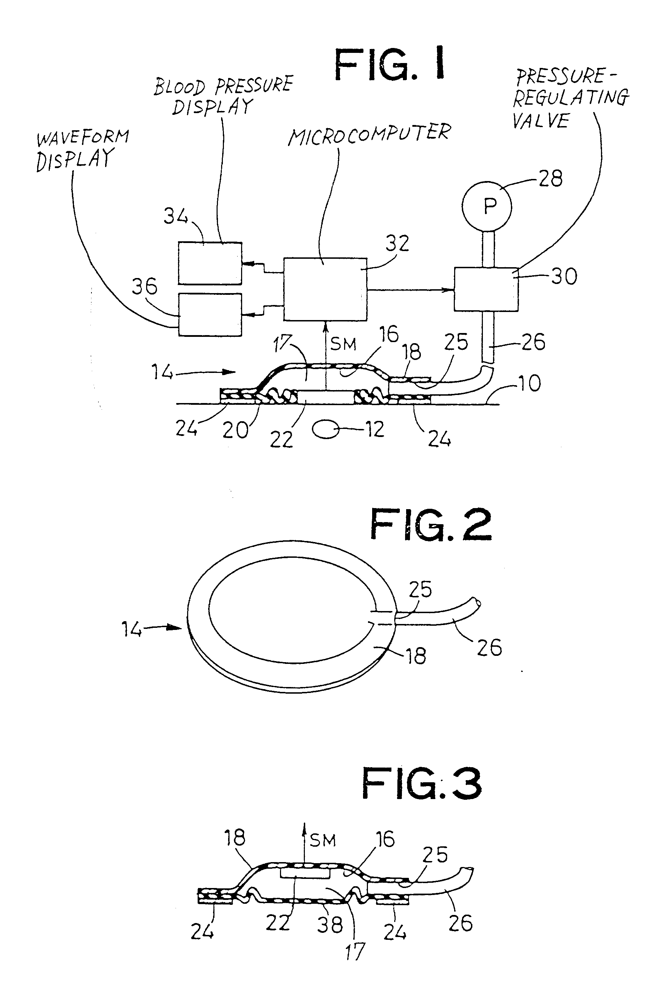

Referring first to Fig. 1, there is shown a pulse wave detecting apparatus embodying the present invention. In the figure, reference numeral 10 designates a body surface of a subject, and reference numeral 12 designates an arterial vessel near the body surface, for example a radial artery. A pulse wave detecting probe 14 is closely adhered to the body surface 10 right above the artery 12.

The pulse wave detecting probe 14 includes a housing 18, a pulse wave sensor 22, a diaphragm 20 and an adhesive sheet 24. The housing 18 has a generally disk-like configuration as shown in Fig. 2, and has a comparatively shallow cavity 16 at a central portion thereof. The housing 18 is formed of an engineering plastic film, and has a comparatively high rigidity. The pulse wave sensor 22 has a strain gauge, piezoelectric element or the like. The diaphragm 20 is fluid-tightly secured at a peripheral portion thereof to a peripheral portion of the disk-like housing 18 along a circumference thereof, and also to a side surface of the pulse wave sensor 22, so that the diaphragm 20 defines a fluid-tight space 17 in the housing 18. The diaphragm 20 is formed of rubber and has a generally annular configuration. The adhesive sheet 24 has a generally annular configuration, and is secured to one surface of the peripheral portion of the diaphragm 20, the other surface of the diaphragm 20 being fixed to the housing 18. The fluid-tight space 17 (shallow cavity 16) is supplied with a pressurized fluid from an electrically operated pump 28 via a pipe 26 inserted in a passage 25 provided at the peripheral portion of the probe 14. Pressure level in the fluid-tight space 17 is adjusted by a pressure-regulating valve 30. The fluid may be either a non-compressive fluid such as water or oil, or a compressive fluid such as air or carbonic-acid gas.

The pulse wave sensor 22 is supported by the diaphragm 20 such that the sensor 22 defines a part of a bottom surface of the pulse wave detecting probe 14 which surface contacts the body surface 10 when the probe 14 is placed on the body surface 10 and to which surface the adhesive sheet 24 is fixed. If the electrically operated pump 28 is operated to supply the fluid-tight space 17 with the pressurized fluid, the diaphragm 20 is inflated and thereby presses the artery 12 via the body surface 10. The pulse wave sensor 22 detects pressure variation produced at an interface between the sensor 22 and the body surface 10, the detected pressure variation representing pulse wave of the artery 12 which is produced in synchronization with heart beat of the subject as a result of the pressing of the diaphragm 20. The sensor 22 generates electrical signal SM representing the thus-detected pulse wave, to a microcomputer 32. In the present embodiment, the diaphragm 20 serves as the pressing means of the pulse wave detecting apparatus, while the pulse wave sensor 22 serves as the pulse wave detecting means.

The microcomputer 32 includes a CPU (central processing unit), a RAM (random access memory), a ROM (read only memory) and an I/O interface (all not shown). The CPU processes the input signal (pulse wave signal SM) according to programs stored in the ROM by utilizing the temporary-storage function of the RAM, and controls the pressure level in the fluid-tight space 17 of the housing 18 through the pressure-regulating valve 30. More specifically described, upon operation of a start switch (not shown), the microcomputer 30 activates the pressure-regulating valve 30 to allow the pressurized fluid from the pump 28 to be supplied to the fluid-tight space 17. Subsequently, the microcomputer 30 calculates the magnitude of the pulse wave signal SM, for example the amplitude or electric power of the signal SM, and thereby determines whether or not the magnitude of the signal SM has been increased to an optimum level, for example whether or not the magnitude has been increased to a maximum level or saturated. When the magnitude of the signal SM has been increased to the optimum level, the microcomputer 32 commands the pressure-regulating valve 30 to maintain the current pressure level in the fluid-tight space 17 of the housing 18 by means of feed-back control. The CPU of the microcomputer 32 determines blood pressure of the subject based on the pulse wave signal SM, and commands a blood pressure display 34 to display the thus-determined blood pressure and concurrently commands a waveform display 36 to display the waveform of the pulse wave represented by the signal SM. Since an upper peak value of the pulse wave corresponds to a maximum blood pressure and a lower peak value of the pulse wave corresponds to a minimum blood pressure, actual blood pressure indicated by the blood pressure display 36 is determined according to a pre-determined relationship between the pulse wave and the blood pressure, and based on the actual upper and lower peak values of the pulse wave represented by the signal SM. Further, the waveform of the pulse wave indicated by the waveform display 36 represents variation in pulse pressure of the artery 12, and provides clinical or medical important information. One of the blood pressure display 34 and the waveform display 36 may be omitted.

There will be described below the operation of the instant pulse wave detecting apparatus. Initially, the pulse wave detecting probe 14 is fixed to the body surface 10 by closely adhering the adhesive sheet 24 secured to the housing 18 to the body surface 10 around right above the artery 12, such that the pulse wave sensor 22 is positioned right above the artery 12. Subsequently the fluid-tight space 17 is supplied with the pressurized fluid from the electrically operated pump 28, and the diaphragm 20 and sensor 22 are pressed against the artery 12 via the body surface 10. Thus, the pulse wave of the artery 12 is detected by the pulse wave sensor 22, which generates the pulse wave signal SM to the microcomputer 32. When the magnitude of the signal SM has come to an optimum level, for example when the magnitude has been increased to a maximum level or saturated, the microcomputer 32 (CPU) commands the pressure-regulating valve 30 to maintain the pressure level in the fluid-tight space 17 of the housing 18. Based on the pulse wave signal SM having the optimum magnitude, the microcomputer 32 determines the blood pressure of the subject and commands the devices 34, 36 to display the thus-determined blood pressure and waveform, respectively. The pulse wave detecting probe 14 (housing 18) is adhered to the body surface 10 by the adhesive sheet 24, with an adhesive force higher than a maximum pressing force of the diaphram 20.

As is apparent from the foregoing, in the instant pulse wave detecting apparatus, the pulse wave sensor 22 is easily fixed to the body surface 10 over the artery 12 by simply adhering the adhesive sheet 24 to the body surface 10 around right above the artery 12 so that the adhesive sheet 24 is brought into close contact with the body surface. Thus, the instant apparatus is more easily handled than the coventional apparatus whose pulse wave sensor is adapted to be fixed on the body surface by winding and fastening a band or similar fixing means around a body member of a subject.

While the present invention has been described in its preferred embodiment, it is to be understood that the invention may be embodied with various modifications. There will be some modified pulse wave detecting probes. The same numerals as used in Figs. 1 and 2 are used to designate the corresponding elements in Figs. 3 through 7, and repetitive description of those elements are omitted.

While in the above-described embodiment the pulse wave sensor 22 is adapted to be directly pressed against the body surface 10, it is possible as shown in Fig. 3 to secure the sensor 22 to the wall of the housing 18 which wall defines the shallow cavity 16 and dispose a diaphragm 38 so as to define a fluid-tight space 17 in the housing 18. In this modified form, the sensor 22 detects pressure variation which is produced in the space 17 by oscillatory pressure wave transmitted from the artery 12 through the diaphragm 38 and the fluid in the space 17. The detected pressure variation represents pulse wave of the artery 12. The diaphragm 38 serves as the pressing means of the pulse wave detecting apparatus.

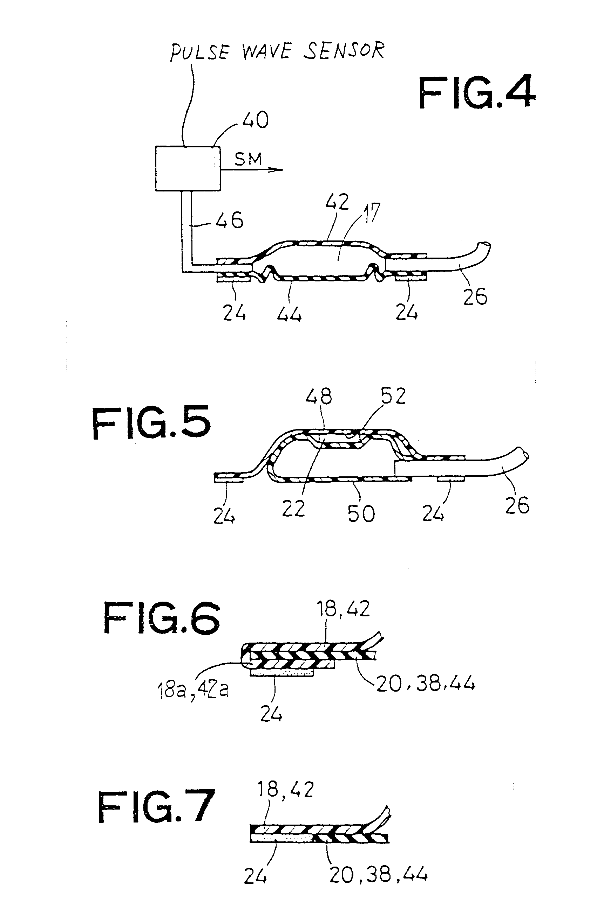

Another modified pulse detecting probe is shown in Fig. 4. In the figure, a pulse wave sensor 40 serving as the pulse wave detecting means is disposed outside a housing 42. A diaphragm 44 serving as the pressing means is secured to the housing 42 to define a fluid-tight space 17, which communicates the pulse wave sensor 40 via a pipe 46. The sensor 40 detects pressure variation (pressure oscillation) in the fluid-tight space 17 in the housing 18. The detected pressure variation represents pulse wave of the artery 12. In this modified form, it is recommended that the pressurized fluid used to inflate the diaphragm 44 be a non-compressible fluid.

Yet another modified pulse detecting probe is shown in Fig. 5. In a housing 48 of the probe, a bag 50 formed of a thin, synthetic resin film is accommodated such that the bag 50 is secured at its opening to the pipe 26 from which a pressurized fluid is supplied thereto. The housing 50 has a cavity 52 in which the bag 50 is inflated by the fluid pressure applied thereto. With the bag 50 inflated in the cavity 52, the pulse wave sensor 22 secured to the wall of the housing 48 is brought into close contact with the inflated bag 50. Thus, the sensor 22 detects pressure variation produced in the bag 50 by oscillatory pressure wave transmitted from the artery 12 through the thin films of the bag 50 and the fluid in the bag 50. The detected pressure variation represents pulse wave of the artery 12. In the present modified form, the bag 50 serves as the pressing means. It is preferred that the bag 50 be high in flexibility and be low in elasticity.

While in the illustrated pulse wave detecting probe of Figs. 1, 3 and 4 the adhesive sheet 24 is secured to the diaphragm 20, 38 and 40, respectively, it is possible to secure the adhesive sheet 24 to the housing 18, 42 as shown in Figs. 6 and 7. In this case, however, a portion of the housing 18, 42 where the pipe 26, 46 is inserted, is free from the adhesive sheet 24. In the arrangement shown in Fig. 6, a portion of the housing 18, 42 where the pipe 26, 46 is inserted, is free from a folded portion 18a, 42a thereof.

## Claims

1. A pulse wave detecting apparatus having
a housing (18, 42, 48) which is to be fixed to a body surface (10) of a subject,
pressing means (20, 38, 44, 50) accommodated in said housing, for pressing an artery (12) of said subject via said body surface, and
pulse wave detecting means (22, 40) for detecting pulse waves of said artery which are produced in relation with the pressing of said pressing means,
characterised by,
an adhesive member (24) mounted on said housing (18, 42, 48) such that said adhesive member is located around said pressing means (20, 38, 44, 50) accommodated in said housing, said adhesive member adhering said housing to said body surface (10) of said subject with an adhesive force higher than the pressing force of said pressing means.

2. The apparatus as set forth in claim 1, wherein said pressing means includes a diaphragm (20) which is inflatable by fluid pressure applied thereto, and
said pulse wave detecting means comprises a pressure sensor (20) which is accommodated in said housing (18) and is pressed against said artery (12) via said body surface (10) by the inflated diaphragm so as to detect pressure variation produced at an interface between said pressure sensor and said body surface, the detected pressure variation representing the pulse wave produced in relation with the pressing of said diaphragm.

3. The apparatus as set forth in claim 1, wherein said pressing means consists of a diaphragm (38) which is inflatable by fluid pressure applied thereto, and said pulse wave detecting means (22, 40) detects pressure variation produced in a space (17) defined by at least said diaphragm, the detected pressure variation representing said pulse wave.

4. The apparatus as set forth in claim 3, wherein said pulse wave detecting means (22) is accommodated in said housing (18).

5. The apparatus as set forth in claim 3, wherein said pulse wave detecting means (40) is located outside said housing (42), and connected to said housing via a pipe (46).

6. The apparatus as set forth in claim 1, wherein said pressing means consists of a bag (50) which is inflated by fluid pressure applied thereto, and said pulse wave detecting means (22) detects pressure variation in said bag, the detected pressure variation representing said pulse wave.

7. The apparatus as set forth in any of claims 1-6, wherein said housing (18, 42, 48) is formed of synthetic resin and has a generally disk-like configuration with a comparatively shallow cavity, and said adhesive member consists of an adhesive sheet (24) fixed to said disk-like housing along a circumference of said housing, said adhesive sheet being adhered to said body surface (10) with said adhesive force.

8. The apparatus as set forth in claim 1, wherein said adhesive member (24) has a generally annular configuration.

## Patentansprüche

1. Pulswellenerfassungsvorrichtung mit
einem Gehäuse (18, 42, 48) zum Anbringen an einer Körperoberfläche (10) eines Patienten,
Druckmitteln (20, 38, 44, 50), die in dem Gehäuse untergebracht sind, um eine Arterie (12) des Patienten über die Körperoberfläche zu drücken, und
Pulswellenerfassungsmitteln (22, 40) zur Erfassung der Pulswellen dieser Arterie, die in Relation zu dem Drücken des Druckmittels erzeugt werden,
gekennzeichnet durch
ein Haftelement (24), das auf dem Gehäuse (18, 42, 48) derart angebracht ist, daß das Haftelement um die in dem Gehäuse untergebrachten Druckmittel (20, 38, 44, 50) herum angeordnet ist, wobei das Haftelement das Gehäuse an der Körperoberfläche (10) des Patienten mit einer Adhäsionskraft anklebt, die höher als die Druckkraft der Druckmittel ist.

2. Vorrichtung nach Anspruch 1, bei der das Druckmittel eine Membran (20) umfaßt, die durch daran angelegten Fluiddruck aufgeblasen werden kann, und
wobei das Pulswellenerfassungsmittel einen Drucksensor (20) umfaßt, der in dem Gehäuse (18) untergebracht ist und gegen die Arterie (12) über die Körperoberfläche (10) durch die aufgeblasene Membran gedrückt wird, um so eine Druckänderung zu erfassen, die an einer Schnittstelle zwischen dem Drucksensor und der Körperoberfläche erzeugt wird, wobei die erfaßte Druckänderung die Pulswelle darstellt, die in Relation zu dem Drücken der Membran erzeugt worden ist.

3. Vorrichtung nach Anspruch 1, bei der das Druckmittel aus einer Membran (38) besteht, die mittels eines daran angelegten Fluiddruckes aufgeblasen werden kann, und wobei das Pulswellenerfassungsmittel (22, 40) eine Druckänderung erfaßt, die in einem Raum (17) erzeugt worden ist, der von zumindest dieser Membran begrenzt wird, wobei die erfaßte Druckänderung die Pulswelle darstellt.

4. Vorrichtung nach Anspruch 3, bei der das Pulswellenerfassungsmittel (22) in dem Gehäuse (18) untergebracht ist.

5. Vorrichtung nach Anspruch 3, bei der sich das Pulswellenerfassungsmittel (40) außerhalb des Gehäuses (42) befindet und mit dem Gehäuse über ein Rohr (46) verbunden ist.

6. Vorrichtung nach Anspruch 1, bei der das Druckmittel aus einem Beutel (50) besteht, der mittels eines daran angelegten Fluiddruckes aufgeblasen werden kann, und bei der das Druckwellenerfassungsmittel (22) eine Druckänderung erfaßt, wobei die erfaßte Druckänderung die Pulswelle darstellt.

7. Vorrichtung nach einem der Ansprüche 1 bis 6, bei der das Gehäuse (18, 42, 48) aus einem Kunstharz gebildet ist und eine im allgemeinen scheibenartige Konfiguration mit einer verhältnismäßig flachen Höhlung aufweist, und bei der das Haftelement aus einer Klebeschicht (24) besteht, die an dem scheibenartigen Gehäuse entlang eines Umfangs des Gehäuses angebracht ist, wobei die Klebeschicht mit der Adhäsionskraft an der Körperoberfläche (10) angeklebt wird.

8. Vorrichtung nach Anspruch 1, bei der das Haftelement (24) eine im allgemeinen ringförmige Konfiguration aufweist.

## Revendications

1. Dispositif pour détecter l'onde à impulsion comprenant
un logement (18, 42, 48) à fixer sur une surface du corps (10) d'un sujet,
un moyen de pression (20, 38, 44, 50) logé dans ledit logement, pour appuyer sur une artère (12) dudit sujet à travers ladite surface du corps, et
un moyen de détection d'onde à impulsion (22, 40) pour détecter les ondes à impulsion de ladite artère, qui sont générées du fait de la pression exercée par ledit moyen de pression,
caractérisé en ce qu'il comprend
un élément adhésif (24) monté sur ledit logement (18, 42, 48) de telle sorte que ledit élément adhésif est situé autour dudit moyen de pression (20, 38, 44, 50) logé dans ledit logement, ledit élément adhésif collant ledit logement sur ladite surface du corps (10) dudit sujet, avec une force adhésive supérieure à la force de pression exercée par ledit moyen de pression.

2. Dispositif selon la revendication 1, caractérisé en ce que ledit moyen de pression comporte une membrane (20) expansible sous la pression d'un fluide, et en ce que ledit moyen de détection d'onde à impulsion comporte un capteur de pression (22) logé dans ledit logement (18) et plaqué contre ladite artère (12) à travers ladite surface de corps (10) par la membrane gonflée, de manière à détecter toute variation de pression générée à une interface entre ledit capteur de pression et ladite surface du corps, la variation de pression détectée représentant l'onde à impulsion générée du fait de la pression exercée par ladite membrane.

3. Dispositif selon la revendication 1, caractérisé en ce que ledit moyen de pression consiste en une membrane (38) expansible sous la pression d'un fluide, et en ce que ledit moyen de détection d'onde à impulsion (22, 40) détecte la variation de pression générée dans un espace (17) défini par au moins ladite membrane, la variation de pression détectée représentant ladite onde à impulsion.

4. Dispositif selon la revendication 3, caractérisé en ce que le moyen de détection d'onde à impulsion (22) est logé dans ledit logement (18).

5. Dispositif selon la revendication 3, caractérisé en ce que ledit moyen de détection d'onde à impulsion (40) est logé à l'extérieur dudit logement (42), et est relié audit logement via un tube (46).

6. Dispositif selon la revendication 1, caractérisé en ce que ledit moyen de pression consiste en un sac (50) gonflé sous la pression d'un fluide, et en ce que ledit moyen de détection d'onde à impulsion (22) détecte la variation de pression dans ledit sac, la variation de pression détectée représentant ladite onde à impulsion.

7. Dispositif selon l'une quelconque des revendications 1 à 6, caractérisé en ce que ledit logement (18, 42, 48) est en résine synthétique et présente une configuration essentiellement en forme de disque avec une cavité relativement peu profonde, et en ce que ledit élément adhésif consiste en une feuille adhésive (24) fixée audit logement en forme de disque, le long d'une circonférence dudit logement, ladite feuille adhésive étant collée à ladite surface de corps (10) avec ladite force adhésive.

8. Dispositif selon la revendication 1, caractérisé en ce que l'élément adhésif (24) a une configuration essentiellement annulaire.
